# EUROPÄISCHE PATENTSCHRIFT

(11) **EP 1 031 355 B1**
(45) Veröffentlichungstag und Bekanntmachung des Hinweises auf die Patenterteilung: **25.08.2004**
(21) Anmeldenummer: 00101150.1
(22) Anmeldetag: 21.01.2000
(51) Int. Cl.: A61L 2/26, D06F 39/00, A47L 15/42

(54) **Vorrichtung zur Kontrolle des Reinigungsergebnisses von Spülmaschinen, insbesondere von Desinfektionsspülmaschinen für medizinische Geräte**
Device for monitoring cleaning results in washing machines, especially machines for washing and disinfecting medical equipment
Dispositif pour contrôler les résultats du nettoyage dans les machines à laver, notamment dans les machines de nettoyage et désinfection pour les instruments médicaux

(30) Priorität: 22.02.1999 DE 29903174 U
(43) Veröffentlichungstag der Anmeldung: 30.08.2000
(73) Patentinhaber: BHT Hygienetechnik GmbH, 86316 Friedberg/Derching (DE)
(72) Erfinder: Püttmann, Matthias, 58239 Schwerte (DE); Büchel, Wolfgang, 41464 Neuss (DE)
(74) Vertreter: von Bülow, Tam, Dr.

(56) Entgegenhaltungen:
- WO-A-98/45470

## Beschreibung

Die Erfindung bezieht sich auf eine Vorrichtung zur Kontrolle des Reinigungsergebnisses von Spülmaschinen, insbesondere von Desinfektionsspülmaschinen für medizinische Geräte.

Bei der Reinigung medizinischer Geräte und Instrumente, aber auch von Gegenständen der industriellen Fertigung, wie z. B. Keramikträger für elektronische Schaltungen usw., ist es vielfach erwünscht, eine Qualitätskontrolle des Reinigungsergebnisses durchzuführen, ohne daß die gereinigten Gegenstände selbst untersucht werden, da die üblichen Kontrollverfahren das Reinigungsergebnis zunichte machen. Die Kontrolle bei medizinischen Geräten besteht nämlich beispielsweise darin, den Gegenstand in eine Nährlösung für Mikroben und Bakterien zu legen und zu beobachten, ob an dem gereinigten Gegenstand noch vorhandene Keime sich vermehren.

Bisher hat man sich damit beholfen, zusätzlich zu den zu reinigenden Gegenständen einen oder mehrere Prüfkörper in die Spülmaschine einzulegen und zu reinigen, wobei dieser Prüfkörper zuvor gezielt mit Bakterienkulturen und anderen Fremdkörpern verunreinigt wurde.

Diese Vorgehensweise ist in mehrfacher Hinsicht problematisch:
- obwohl für die gezielte Verunreinigung der Prüfkörper weitgehend ungefährliche Bakterienkulturen verwendet werden, besteht die Gefahr, daß auf dem Weg von der "Verunreinigungsstation" zur Spülmaschine diese Bakterienkulturen auch die Umwelt kontaminieren;
- umgekehrt besteht bei der Entnahme der Prüfkörper aus der Spülmaschine die Gefahr, daß bei nicht einwandfreiem Reinigungsergebnis der noch kontaminierte Prüfkörper auf dem Weg zur Prüfstation ebenfalls die Umwelt kontaminiert;
- schließlich besteht die Gefahr, daß der Prüfkörper trotz einwandfreier Reinigung und Desinfektion in der Spülmaschine auf dem Weg zur Prüfstation aus der Umwelt kontaminiert wird und dann die Prüfstation ein ungenügendes Reinigungsergebnis feststellt, obwohl die Reinigung einwandfrei war.

Mit der Erfindung sollen diese Nachteile beseitigt werden. Aufgabe der Erfindung ist es daher, eine Vorrichtung der eingangs genannten Art zu schaffen, mit der eine korrekte Überprüfung des Reinigungsergebnisses einer Desinfektionsspülmaschine möglich ist und mit der verhindert wird, daß zwischen einem Prüfkörper und der Umwelt eine wechselseitige Kontamination stattfinden kann.

Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Das Grundprinzip der Erfindung besteht darin, daß der bzw. die Prüfkörper in einem weitgehend geschlossenen System zur Spülmaschine und von der Spülmaschine zur Prüfstation verbracht werden und auch während der Reinigung in diesem weitestgehend geschlossenen System verbleiben.

Die Vorrichtung enthält im Prinzip mindestens drei Teile, nämlich ein Kopfteil, das mindestens eine Öffnung für die Zufuhr von Reinigungsflüssigkeit aufweist, mindestens ein an das Kopfteil ankuppelbares Zwischenteil, das eine Prüfkammer zur Aufnahme des Prüfkörpers aufweist, und ein Endteil, das an das Zwischenteil ankuppelbar ist und eine Auslaßöffnung für die Reinigungsflüssigkeit aufweist.

Durch die Kuppelbarkeit dieser Teile können beliebig viele Zwischenteile eingesetzt werden.

Jedes Zwischenteil ist durch einen Siebeinsatz zum benachbarten Teil abgetrennt, so daß der Prüfkörper in "seiner" Prüfkammer verbleibt, die Reinigungsflüssigkeit aber die Vorrichtung durchströmen kann. Auch das Endteil hat einen entsprechenden Siebeinsatz, so daß die dem Endteil nächstgelegene Prüfkammer beidseitig durch einen Siebeinsatz in dem Sinne abgeschlossen ist, daß der Prüfkörper in der Prüfkammer gehalten ist.

Die einzelnen Teile werden vorzugsweise durch eine Bajonettkupplung miteinander gekoppelt. Auch andere Kupplungsarten, wie z. B. eine Schraubkupplung, sind möglich.

Als Prüfkörper werden vorzugsweise eine Gewindeschraube und/oder ein Schlauchstück verwendet. Eine Gewindeschraube eignet sich hervorragend als Prüfkörper, da in den einzelnen Gewindegängen Verunreinigungen und Bakterien sehr gut haften und eine einwandfreie Reinigung an der Gewindeschraube ein sicheres Indiz dafür ist, daß auch komplizierte medizinische Geräte einwandfrei gereinigt werden. In ähnlicher Weise ist ein Schlauchabschnitt ein hervorragend geeigneter Prüfkörper, da er Hohlräume simuliert, wie z. B. Kanäle von Endoskopen.

Um sicher zu stellen, daß der Prüfkörper "Schlauchstück" einwandfrei in der Prüfkammer gehalten ist und nicht an deren Wandungen anliegt, ist an dem Siebeinsatz dessen Prüfkammer ein Zentrierdorn vorgesehen. In ähnlicher Weise kann an dem angekoppelten Gegenstück, wie z. B. dem Endteil, ein Zentrierdorn vorhanden sein.

Das Kopfteil hat vorzugsweise mehrere Öffnungen in Form von Luer-Lock-Anschlüssen, wodurch die Vorrichtung an die verschiedensten Spülmaschinen adaptierbar ist. An einen oder mehrere dieser Luer-Lock-Anschlüsse kann ein Schlauch angeschlossen werden, über den direkt Reinigungsflüssigkeit, Trocknungsluft etc. durch die Vorrichtung geleitet werden kann. Nicht benötigte Luer-Lock-Anschlüsse können durch Blindstopfen verschlossen werden. Auch kann bei manueller Reinigung an den Luer-Lock-Anschluß eine Spritze angeschlossen werden, ähnlich wie es bei der manuellen Reinigung von Endoskopen mit den Kanälen des Endoskopes geschieht. Wird die Vorrichtung in einer Desinfektionsspülmaschine mit Druckkammer eingesetzt, wie sie beispielsweise in der DE-C1-38 19 257 oder der DE-U1-93 14 488 beschrieben ist, so können alle Luer-Lock-Anschlüsse zur Druckkammer hin offen sein und aus dieser gespeist werden.

Für die Verwendung der Vorrichtung in einer solchen Maschine mit Druckkammer ist zusätzlich vorgesehen, daß zwischen dem Endteil und dem unmittelbaren anschließenden Zwischenteil eine Anschlagplatte angeordnet ist, die die Vorrichtung in der Druckkammer hält. Das Endteil kann darüber hinaus zusätzlich einen Anschlußstutzen für einen Schlauchabschnitt aufweisen, der die Funktion des Schlauches der DE-C1-38 19 257 nachbildet.

Im folgenden wird die Erfindung anhand eines Ausführungsbeispieles im Zusammenhang mit der Zeichnung ausführlicher erläutert. Es zeigt:

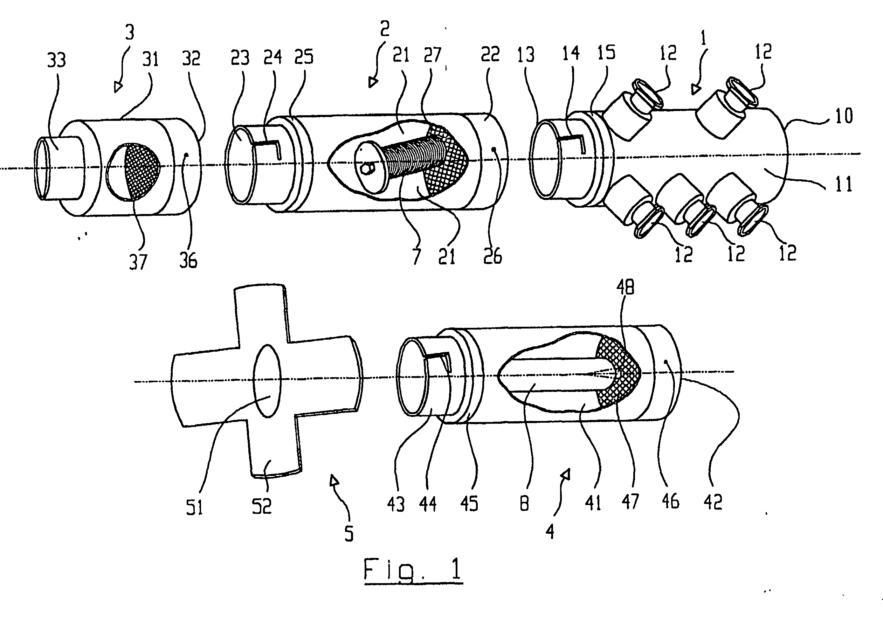

- Fig. 1: eine perspektivische Explosionsdarstellung von Einzelteilen der Vorrichtung nach der Erfindung; und
- Fig. 2: eine perspektivische, teilweise aufgeschnittene Ansicht einer zusammengesetzten Vorrichtung nach der Erfindung.

Gleiche Bezugszeichen in den einzelnen Figuren bezeichnen gleiche bzw. funktionell einander entsprechende Teile.

Die Vorrichtung nach der Erfindung hat mindestens drei Komponenten, nämlich ein Kopfteil 1, ein Zwischenteil 2 und ein Endteil 3. Das Kopfteil 1 besteht im Prinzip aus einem hohlzylindrischen Körper 11, dessen eines Ende durch eine Endwand 10 verschlossen ist. Der Außenmantel des Körpers 11 ist durch mehrere Luer-Lock-Anschlüsse 12 durchbrochen, durch welche von außen ein Zugang zum Inneren des Hohlkörpers 11 möglich ist. Das der Endwand 10 gegenüberliegende Ende des hohlzylindrischen Körpers 11 weist einen Anschlußstutzen 13 auf, der einen Bajonettschlitz 14 besitzt. Der Durchmesser des Anschlußstutzens 13 ist geringer als der Außendurchmesser des hohlzylindrischen Körpers 11, so daß im Fußbereich des Anschlußstutzens 13 eine Stufe gebildet ist, an der ein Dichtungsring 15 abgestützt ist. Das erste Zwischenteil 2 ist ebenfalls als Hohlzylinder ausgebildet und besitzt eine zylindrische Prüfkammer 21, in die ein Prüfkörper, wie hier eine Schraube 7, eingesetzt werden kann. Ein Ende des Zwischenteiles 2 hat eine Muffe 22, deren Innendurchmesser an den Anschlußstutzen 13 des Kopfteiles angepaßt ist. Weiter ist an der Muffe 22 ein radial nach innen ragender Bajonettstift 26 vorgesehen, der mit dem Bajonettschlitz 14 des Kopfteiles zusammenpaßt. Somit können das Kopfteil 1 und das Zwischenteil 2 aneinander gekuppelt werden, so daß deren innere Hohlräume in Strömungsverbindung miteinander stehen. Die Prüfkammer 21 ist in Richtung zur Muffe 22 durch einen Siebeinsatz 27 abgetrennt. Durch den Siebeinsatz wird einerseits der Prüfkörper gegen ein Herausfallen in Richtung zum Kopfteil gesichert und andererseits wird durch das Sieb eine gleichmäßigere Strömung von Reinigungsflüssigkeit erreicht.

Ähnlich wie das Kopfteil 1 hat auch das Zwischenteil 2 einen maßgleichen Anschlußstutzen 23 mit Bajonettschlitz 24, wobei auch hier eine Ringdichtung 25 im Fußbereich des Anschlußstutzens 23 vorgesehen sein kann. Damit ist das Zwischenteil 2 an ein weiteres Zwischenteil, wie z. B. das Zwischenteil 4, oder an ein Endteil 3 ankuppelbar. Das Endteil 3 oder auch jedes weitere Zwischenteil 4 hat dementsprechend eine Muffe 32 (bzw. 42) mit Bajonettstift 36 (bzw. 46) und wiederum einen Siebeinsatz 37 (bzw. 47) im hohlzylindrischen Innenraum 31 (bzw. der Prüfkammer 41).

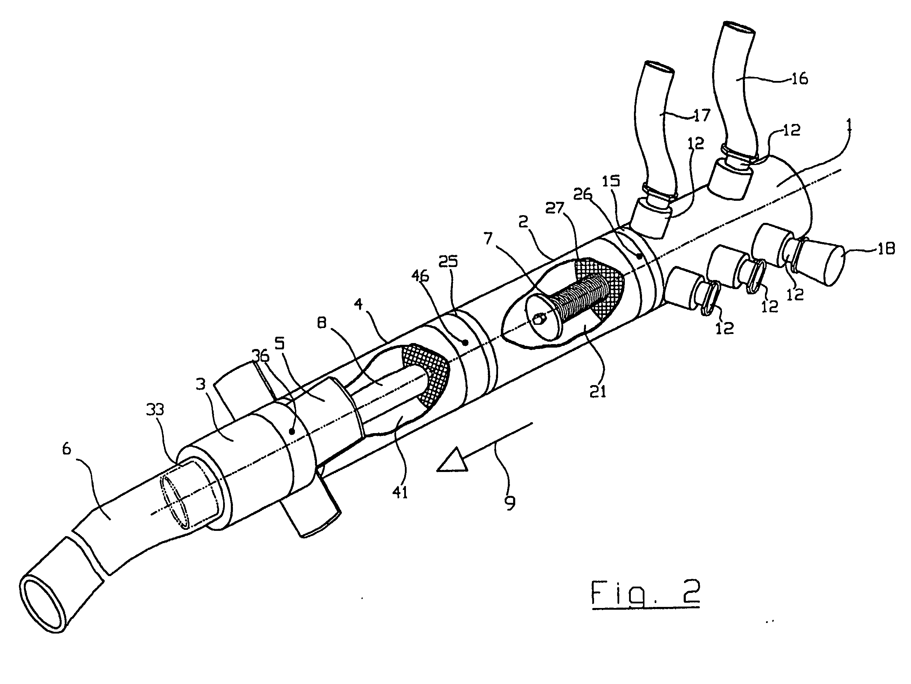

Das Endteil 3 hat austrittsseitig einen Anschlußstutzen 33, dessen Durchmesser ebenfalls geringer ist als der Außendurchmesser des hohlzylindrischen Endteiles. Aus diesem Anschlußstutzen kann die Reinigungsflüssigkeit die Gesamtvorrichtung verlassen. Auch kann an diesen Anschlußstutzen 33 ein Schlauchabschnitt aufgesteckt werden (vgl. Schlauchabschnitt 6 in Fig. 2), dessen Funktion oben erläutert wurde.

Das zweite Zwischenteil 4 hat wiederum einen identischen Anschlußstutzen wie das erste Zwischenteil, d. h. den Anschlußstutzen 43 mit Bajonettschlitz 44 und einem Dichtungsring 45.

Die Prüfkammer 41 des zweiten Zwischenteiles ist hier spezieller für einen Prüfkörper 8 ausgebildet, der ein Schlauchabschnitt ist. Zu diesem Zwecke ist an dem Siebeinsatz 47 ein Zentrierdorn 48 vorgesehen, auf den der Schlauchabschnitt aufgesteckt werden kann. Dadurch ist sichergestellt, daß der Schlauchabschnitt an der Strömungseinlaßseite nicht an der Innenwandung der Prüfkammer 41 anliegt.

Da die Anschlüsse der Zwischenteile jeweils gleich sind, können im Prinzip beliebig viele solcher Zwischenteile aneinander gekuppelt werden. Das letzte Zwischenteil wird dann mit dem Endteil 3 verbunden.

Schließlich kann zwischen das letzte Zwischenteil und das Endteil noch eine Anschlagplatte 5 eingesetzt werden, die eine mittige Öffnung 51 entsprechend dem Außendurchmesser der Anschlußstutzen 13, 23, 43 aufweist und die über die Außenkontur (Durchmesser) der Einzelteile herausragt, was hier mit vier rechtwinklig zueinander stehenden Armen 52 erfolgt. Mit dieser Anschlagplatte kann die gesamte Vorrichtung in einer Druckkammer einer Desinfektionsspülmaschine gehalten werden.

Die Handhabung der Vorrichtung ist wie folgt:

An einer Station, die durchaus entfernt von der Desinfektionsspülmaschine gelegen sein kann, werden die Prüfkörper gezielt mit Bakterienkulturen, sonstigen Keimen und Verunreinigungen kontaminiert und in die entsprechende Prüfkammer (21, 41) des jeweiligen Zwischenteiles (2, 4) eingesetzt. Sodann wird die Vorrichtung insgesamt zusammengesetzt, d. h. eventuell vorhandene mehrere Zwischenteile werden aneinander gekuppelt und Kopfteil 1 und Endteil 3 werden an die jeweiligen Enden angekuppelt. Gegebenenfalls können noch alle Luer-Lock-Anschlüsse durch Blindstopfen verschlossen werden. Schließlich kann auch noch das Endrohr 33 durch einen Blindstopfen verschlossen werden. Die Prüfkörper sind dann in einem geschlossenen System untergebracht und können weder die Umwelt kontaminieren noch aus der Umwelt kontaminiert werden. Die Vorrichtung wird dann zur Desinfektionsspülmaschine verbracht und je nach Art der verwendeten Maschine angeschlossen. So werden beispielsweise Schläuche (vgl. 16 und 17 in Fig. 2) an die Luer-Lock-Anschlüsse angeschlossen, über die Reinigungsflüssigkeit, Trocknungsluft etc. der Vorrichtung zugeführt werden. Gegebenenfalls wird auch noch der Schlauch 6 angeschlossen. Nach Abschluß des Reinigungsvorganges wird die Vorrichtung mit den gereinigten Prüfkörpern zur Prüfstation verbracht und abschnittweise entkuppelt, wobei die jeweiligen Prüfkörper ohne Berührung durch einen Menschen in die Nährlösung verbracht werden, allein durch Auskippen, d. h. durch Schwerkraft. Dabei ist ausgeschlossen, daß Fremdkeime aus der Umgebung mit eingeschleppt werden. Umgekehrt ist ebenfalls sichergestellt, daß während des Transportes eine wechselseitige Kontamination stattfindet.

In Fig. 2 ist die Vorrichtung im zusammengebauten Zustand dargestellt, wobei hier zwei Zwischenstücke 2 und 4 verwendet werden. Die Hauptströmungsrichtung der Reinigungsflüssigkeit ist durch den Pfeil 9 dargestellt. Ein Luer-Lock-Anschluß ist durch einen Verschlußstopfen 18 geschlossen. Selbstverständlich können beliebige solcher Verschlußstopfen verwendet werden. An zwei weitere Luer-Lock-Anschlüsse sind zwei Schläuche 16 und 17 angeschlossen. Auch ist an den Endanschluß 33 ein Schlauch 6 angeschlossen. Schließlich ist auch zu erkennen, daß die Anschlagplatte 5 zwischen dem Endteil 3 und dem dazu nächstgelegenen Zwischenteil 4 angeordnet ist.

## Patentansprüche

1. Vorrichtung zur Kontrolle des Reinigungsergebnisses von Spülmaschinen, insbesondere von Desinfektionsspülmaschinen für medizinische Geräte, **gekennzeichnet durch**
- ein Kopfteil (1), das mindestens eine Öffnung (12) für die Zufuhr von Reinigungsflüssigkeit aufweist,
- mindestens ein an das Kopfteil (1) ankuppelbares Zwischenteil (2, 4), das eine Prüfkammer (21, 41) zur Aufnahme eines Prüfkörpers (7, 8) aufweist und
- ein Endteil (3), das an das Zwischenteil (2, 4) ankuppelbar ist und eine Auslaßöffnung (33) für die Reinigungsflüssigkeit aufweist.

2. Vorrichtung nach Anspruch 1, **dadurch gekennzeichnet, daß** das mindestens eine Zwischenteil (2, 4) und das Endteil (3) je einen Siebeinsatz (27, 37, 47) aufweisen.

3. Vorrichtung nach Anspruch 1 oder 2, **dadurch gekennzeichnet,**
**daß** das Kopfteil (1), das mindestens eine Zwischenteil (2, 4) und das Endteil (3) jeweils zusammenpassende Teile einer Bajonettkupplung (13, 14, 22, 26; 23, 24, 32, 36; 43, 44) aufweisen.

4. Vorrichtung nach Anspruch 1 oder 2, **dadurch gekennzeichnet,**
**daß** das Kopfteil (1), das mindestens eine Zwischenteil (2, 4) und das Endteil (3) jeweils zusammenpassende Teile einer Schraubkupplung aufweisen.

5. Vorrichtung nach einem der Ansprüche 1 bis 4, **dadurch gekennzeichnet,**
**daß** der Prüfkörper eine Gewindeschraube (7) ist.

6. Vorrichtung nach einem der Ansprüche 1 bis 4, **dadurch gekennzeichnet,**
**daß** der Prüfkörper ein Schlauchstück (8) ist.

7. Vorrichtung nach Anspruch 6, **dadurch gekennzeichnet, daß** an dem Siebeinsatz (47) desjenigen Zwischenteils (4), das das Schlauchstück (8) aufnimmt, ein Zentrierdorn (48) angebracht ist, der in das Innere der Prüfkammer (41) hineinragt.

8. Vorrichtung nach einem der Ansprüche 1 bis 7, **dadurch gekennzeichnet,**
**daß** das Kopfteil (1) an seiner Mantelfläche mindestens eine Öffnung in Form eines Luer-Lock-Anschlusses (12) aufweist.

9. Vorrichtung nach einem der Ansprüche 1 bis 8, **dadurch gekennzeichnet,**
**daß** zwischen das Endteil (3) und das unmittelbare daran anschließende Zwischenteil (2, 4) eine Anschlagplatte (5) kuppelbar ist, deren Außenkontur über den Durchmesser des Endteiles (3) hinausragt.

## Claims

1. A device for monitoring cleaning results in washing machines, particularly machines for washing and disinfecting medical equipment, **characterised by**
- a head part (1) which has at least one opening (12) for the supply of cleaning fluid,
- at least one intermediate part (2, 4) which may be coupled to the head part (1) and has a test chamber (21, 41) for receiving a test body (7, 8) and
- an end part (3) which may be coupled to the intermediate part (2, 4) and has an outlet opening (33) for the cleaning fluid.

2. A device according to Claim 1, **characterised in that** at least one intermediate part (2, 4) and the end part (3) each have a screen insert (27, 37, 47).

3. A device according to Claim 1 or 2, **characterised in that** the head part (1), the at least one intermediate part (2, 4) and the end part (3) each have mutually matching parts of a bayonet coupling (13, 14, 22, 26; 23, 24, 32, 36; 43, 44).

4. A device according to Claim 1 or 2, **characterised in that** the head part (1), the at least one intermediate part (2, 4) and the end part (3) each have mutually matching parts of a screw coupling.

5. A device according to one of Claims 1 to 4, **characterised in that** the test body is a threaded screw (7).

6. A device according to one of Claims 1 to 4, **characterised in that** the test body is a hose piece (8).

7. A device according to Claim 6, **characterised in that**, mounted on the screen insert (47) of that intermediate part (4) which receives the hose piece (8), there is a centring mandrel (48) which projects inside the test chamber (41).

8. A device according to one of Claims 1 to 7, **characterised in that** the head part (1) has at least one opening in the form of a Luer lock connection (12) on its lateral surface.

9. A device according to one of Claims 1 to 8, **characterised in that** a stop plate (5) whereof the outer contour projects beyond the diameter of the end part (3) may be coupled between the end part (3) and the intermediate part (2, 4) immediately adjacent thereto.

## Revendications

1. Dispositif pour contrôler les résultats du nettoyage dans les machines à laver, notamment dans les machines de nettoyage et désinfection pour les instruments médicaux, **caractérisé par**
- une pièce de tête (1), qui présente au moins un orifice (12) pour l'apport de liquide de nettoyage,
- au moins une pièce intermédiaire (2, 4) accouplable à la pièce de tête (1), présentant une chambre d'essais (21, 41) destinée à recevoir un échantillon (7, 8), et
- une pièce terminale (3), accouplable à la pièce intermédiaire (2, 4) et présentant un orifice de sortie (33) pour le liquide de nettoyage.

2. Dispositif selon la revendication 1, **caractérisé en ce que**, ladite au moins une pièce intermédiaire (2, 4) et la pièce terminale (3) présentent respectivement une plaque-filtre (27, 37, 47).

3. Dispositif selon l'une des revendications 1 ou 2, **caractérisé en ce que**,
la pièce de tête (1), ladite au moins une pièce intermédiaire (2, 4) et la pièce terminale (3) présentent respectivement des pièces compatibles d'un accouplement à baïonnette (13, 14, 22, 26 ; 23, 24, 32, 36 ; 43, 44).

4. Dispositif selon l'une des revendications 1 ou 2, **caractérisé en ce que**,
la pièce de tête (1), ladite au moins une pièce intermédiaire (2, 4) et la pièce terminale (3) présentent respectivement des pièces compatibles d'un accouplement à vis.

5. Dispositif selon l'une des revendications 1 à 4, **caractérisé en ce que**,
l'échantillon est un boulon fileté (7).

6. Dispositif selon l'une des revendications 1 à 4, **caractérisé en ce que**,
l'échantillon est un morceau de tuyau flexible (8).

7. Dispositif selon la revendication 6, **caractérisé en ce que**, sur la plaque-filtre (47) de la pièce intermédiaire (4), qui reçoit le morceau de tuyau flexible (8), est montée une broche de centrage (48), qui dépasse à l'intérieur de la chambre d'essais (41).

8. Dispositif selon l'une des revendications 1 à 7, **caractérisé en ce que**,
la pièce de tête (1) présente sur sa surface latérale au moins un orifice sous forme de raccord Luer Lock (12).

9. Dispositif selon l'une des revendications 1 à 8, **caractérisé en ce que**,
entre la pièce terminale (3) et la pièce intermédiaire (2, 4) directement raccordée à cette dernière, on peut coupler une plaque de butée (5), dont le contour extérieur dépasse par rapport au diamètre de la pièce terminale (3).
